# EUROPEAN PATENT APPLICATION

(11) **EP 3 287 462 A1**
(43) Date of publication of application: **28.02.2018**
(21) Application number: 16185712.3
(22) Date of filing: 25.08.2016
(51) Int. Cl.: C07D 493/04, A61K 31/34

(54) **ISOSORBIDE DERIVATIVES TO TREAT BACTERIAL BIOFILMS**

(71) Applicant: NITTO DENKO CORPORATION, Osaka 567-8680 (JP)
(72) Inventor: Schüwer, Nicolas, 1007 Lausanne (CH); De Visscher, Geofrey, 1071 Chexbres (CH); De Titta, Alexandre, 1028 Préverenges (CH)
(74) Representative: Hoffmann Eitle

(57) **Abstract**

The invention provides compounds according to Formula (1) which are useful in treating bacterial biofilms, as well as compositions comprising compounds according to Formula (1) and one or more antibiotics, as well as medical devices comprising compounds according to Formula (1): 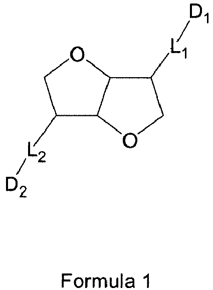 wherein
D₁ and D₂ are independently selected from -H, -OH, -COOR₁, -F, -Cl, Br, -O-C₁-₆-alkyl,-CN, -CF₃, -SH, -NR₁R₂, a C1-C22 alkyl group, a C2-C22 alkenyl group, a C2-C22 alkynyl group, and a 5-10 membered aromatic hetero- or non-hetero mono- or bicyclic group,
wherein the C1-C22 alkyl group, the C2-C22 alkenyl group, and the C2-C22 alkynyl group is optionally substituted with 1 to 5 substituents independently selected from =O, -OH, - COOR₁, -F, -Cl, Br, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O, -OH, - COOR₁, -F, -Cl, Br, -C₁₋₆-alkyl, -C₂₋₆-alkenyl, -O-C₁₋₆-alkyl, -O-C₂₋₆-alkenyl, -CN, -CF₃, -SH and -NR₁R₂,
wherein the 5-10 membered aromatic group is optionally substituted with 1 to 5 substituents independently selected from =O, -OH, -COOR₁, -F, -Cl, -Br, -C₁₋₆-alkyl, -C₂₋₆-alkenyl, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O, -OH, -COOR₁, -F, -Cl, -Br, -C₁₋₆-alkyl, -C₂₋₆-alkenyl, -O-C₁₋₆-alkyl, -O-C₁₋6-alkenyl, -CN, -CF₃, -SH and -NR₁R₂,
wherein R₁ and R₂ in the above definitions are independently selected from H and C₁₋₆-alkyl;
and with the proviso that at least one of D₁ and D₂ is a 5-10 membered aromatic group substituted with one or more -OH groups and/or one or more -COOH groups;
and wherein L₁ and L₂ represent the bonds by which D₁ and D₂ are connected to the tetrahydrofuran rings, with L₁ and L₂ being independently selected from a direct single or direct double bond, an ester bond, an amide bond, an ether bond, a ketoester bond (i.e. *α*-ketoester, *β-*ketoester), a carbamate bond, a carbonate bond, a carbamide bond, a phosphate ester bond, a phosphate amine bond, a phosphonic ester bond, a phosphite ester bond, a phosphonate bond, a phosphinite bond, a phosphorane thiocarbonate bond, a dithiocarbonate bond, a thioether bond, a sulphite bond, a sulfonate bond, a sulfanate bond, a sulfonate bond, a silanol bond, a silyl ether bond, a hydroxylamine bond, an oxime bond, an imine bond, a boronate ester bond and an imino ether bond.

## Description

### 1. FIELD OF THE INVENTION

The invention provides compounds according to Formula (1) which are useful in treating bacterial biofilms, as well as compositions comprising compounds according to Formula (1) and one or more antibiotics, as well as medical devices comprising compounds according to Formula (1).

### 2. BACKGROUND OF THE INVENTION

Bacteria communicate with one another using chemical signal molecules. The information supplied by these chemical signal molecules is critical for synchronizing the activities of large groups of bacterial cells. This chemical communication involves producing, releasing, detecting, and responding to small hormone-like molecules, or autoinducers. This process is otherwise known as quorum sensing, and it allows bacteria to monitor the environment for other bacteria and to alter behaviour on a population-wide scale in response to changes in the number and/or species present in a community. Most quorum sensing-controlled processes are beneficial to the population when carried out simultaneously by a large number of cells. Thus, inhibition of quorum sensing can disrupt the health of a bacterial community, and if the bacteria are pathogenic or responsible for infection, inhibition of quorum sensing can have therapeutic, antibacterial value.

Quorum sensing plays a large role in the formation of biofilms. A biofilm is made up of bacteria, often multi-species, embedded in an extracellular matrix of their own making, and further comprises proteins, polysaccharides and DNA. The build up of the extracellular matrix starts when enough bacteria are present on a surface and quorum sensing mechanisms induces cells to transit from a planktonic to a biofilm state. When in biofilms, the metabolism and the complexity of the environment change, which oftentimes makes it more difficult to treat the bacteria with antibiotics.

Biofilm deposition causes a plethora of problems in industrial, biotechnological and medical areas. In industry, for example, the deposition of biofilm on food preparation and packaging equipment, production lines, etc. can lead to harmful contamination that is difficult to control. For example, *Legionella spp* are known to thrive in warm water and thus pose a concern to public health wherever warm water is used. In the biotechnology sector, clean production methods and purification steps are required and any bacterial contamination can lead to product contamination and loss. In the medical setting, bacterial biofilms play an important role in a number of diseases, including the exacerbation of cystic fibrosis, chronic urinary tract infections, chronic sinus infections, ear infections, acne, dental caries, dental plaque, periodontitis, nosocomial infections, chronic wounds, infections oftentimes due to insertion of medical devices such as catheters, ventilators, prosthetic heart valves, contact lenses, and intrauterine devices.

Antibiotic resistance is rising to dangerously high levels in all parts of the world leading to tougher to treat biofilms, higher medical costs, prolonged hospital stays and increased mortality. There is a demand for compounds that can inhibit biofilm formation, or when used together with antibiotics, potentiate the effect of those antibiotics. The present invention provides such compounds.

### 3. SUMMARY OF THE INVENTION

The inventors have discovered that compounds having two fused tetrahydrofuran rings substituted with at least one aromatic ring having one or more -OH groups and/or one or more-COOH groups will effectively prevent or treat a bacterial biofilm, and furthermore can be used to potentiate an antibiotic. Thus, in one aspect the present invention relates to compounds of Formula (1): 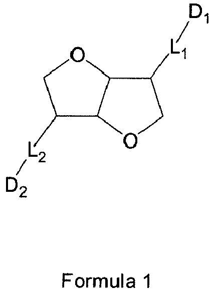 wherein:
D₁ and D₂ are independently selected from -H, -OH, -COOR₁, -F, -Cl, Br, -O-C₁₋₆-alkyl, -CN, -CF₃, -SH, -NR₁R₂, a C1-C22 alkyl group, a C2-C22 alkenyl group, a C2-C22 alkynyl group, and a 5-10 membered aromatic hetero- or non-hetero mono- or bicyclic group,
wherein the C1-C22 alkyl group, the C2-C22 alkenyl group, and the C2-C22 alkynyl group is optionally substituted with 1 to 5 substituents independently selected from =O, -OH, -COOR₁, -F, -Cl, Br, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O,-OH, -COOR₁, -F, -Cl, Br, -C₁₋₆-alkyl, -C₂₋₆-alkenyl, -O-C₁₋₆-alkyl, -O-C₂₋₆-alkenyl, -CN, -CF₃,-SH and -NR₁R₂,
wherein the 5-10 membered aromatic group is optionally substituted with 1 to 5 substituents independently selected from =O, -OH, -COOR₁, -F, -Cl, Br, -C₁₋₆-alkyl, -C₂₋₆-alkenyl, -O-C₁₋₆-alkyl, -O-C₁₋₆-alkenyl, -CN, -CF₃, -SH, -NR₁R₂, and phenyl, wherein said phenyl is optionally substituted with 1 to 3 substituents selected from =O, -OH, -COOR₁, -F, -Cl, Br, -C₁₋₆-alkyl, -C₂₋₆-alkenyl, -O-C₁₋₆-alkyl, -O-C₂₋₆-alkenyl, -CN, -CF₃, -SH and -NR₁R₂,
wherein R₁ and R₂ in the above definitions are independently selected from H and C₁₋₆ alkyl;
and with the proviso that at least one of D₁ and D₂ is a 5-10 membered aromatic group substituted with one or more -OH groups and/or one or more -COOH groups;
L₁ and L₂ represent the bonds by which D₁ and D₂ are connected to the tetrahydrofuran rings, with L₁ and L₂ being independently selected from a direct single or direct double bond, an ester bond, an amide bond, an ether bond, a ketoester bond (i.e. α-ketoester, β-ketoester), a carbamate bond, a carbonate bond, a carbamide bond, a phosphate ester bond, a phosphate amine bond, a phosphonic ester bond, a phosphite ester bond, a phosphonate bond, a phosphinite bond, a phosphorane thiocarbonate bond, a dithiocarbonate bond, a thioether bond, a sulphite bond, a sulfonate bond, a sulfanate bond, a sulfonate bond, a silanol bond, a silyl ether bond, a hydroxylamine bond, an oxime bond, an imine bond, a boronate ester bond and an imino ether bond.

In one aspect the invention relates to compounds according to Formula (1) for use in a therapeutic method of preventing a bacterial biofilm from forming in or on a human or animal, for use in a therapeutic method of hindering the formation of a biofilm in or on a human or animal, and/or for use in a therapeutic method of treating (e.g. reduce or eradicate) a bacterial biofilm that is forming or has already formed in or on a human or animal.

Compounds according to Formula (1) can also be used to treat biofilms on an inanimate substrate. Thus, one aspect of the present invention relates to the use of compounds according to Formula (1) for preventing a bacterial biofilm from forming in or on an inanimate substrate, to hinder the formation of a biofilm in or on an inanimate substrate, and/or to treat (e.g. reduce or eradicate) a bacterial biofilm that is forming or has already formed in or on an inanimate substrate. The substrate can be any surface where it is desirable to prevent a biofilm forming such as on a medical device, on household or industrial machinery, on a wall, floor, bench and the like.

The inventors have also discovered that compounds according to Formula (1) can be used to potentiate the effect of an antibiotic. Thus, in one aspect the invention relates to methods of treating bacterial infections or biofilm where a compound according to Formula (1) is used concomitantly with an antibiotic. For convenience, one or more compounds according to Formula (1) can be formulated together with one or more antibiotics in a single composition.

Compounds according to Formula (1), and compositions comprising compounds according to Formula (1) with one or more antibiotics, can be applied to a medical device to prevent, hinder or otherwise treat a biofilm. In one aspect the invention relates to medical devices comprising such compounds or compositions. In another aspect, the invention relates to medical devices such as an adhesive patch for delivering such compounds and compositions to target site.

### 4. BRIEF DESCRIPTION OF THE DRAWINGS

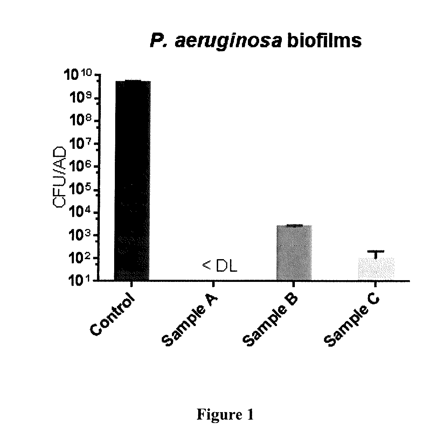

Figure 1 shows that a composition comprising a combination of isosorbide digallate ester and tetracycline (Sample A) has a better biofilm eradicating effect than a composition comprising an equivalent amount of tetracycline alone (Sample B) or a composition comprising equivalent amounts of the individual components tetracycline, isosorbide and gallic acid (Sample C).

### 5. DETAILED DESCRIPTION OF THE INVENTION

### 5.1 General remarks

As used herein, the terms "comprises", "comprising", "includes", "including", "has", "having" or any other variation thereof, are intended to cover a non-exclusive inclusion. That said, the terms "comprises", "comprising", "includes", "including", "has", "having" or any other variation thereof can also be understood to cover the disclosed embodiment having no further additional components *(i.e.* consisting of those components).

Also, the indefinite articles "a" and "an" preceding an element or component of the invention are intended to be non-restrictive regarding the number of instances *(i.e.* occurrences) of the element or component. Therefore "a" or "an" should be read to include one or at least one, and the singular word form of the element or component also includes the plural unless the number is obviously meant to be singular.

Further, when an aspect of the invention is described as being 'preferred', it should be understood that this preferred aspect of the invention can be combined with other preferred aspects of the invention.

### 5.2 Compounds according to Formula (1)

The present invention provides compounds according to Formula (1): 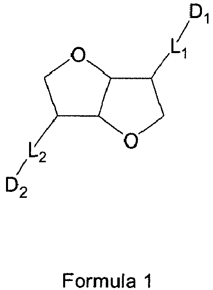 wherein:
D₁ and D₂ are independently selected from -H, -OH, -COOR₁, -F, -Cl, Br, -O-C₁₋₆-alkyl, -CN, -CF₃, -SH, -NR₁R₂, a C1-C22 alkyl group, a C2-C22 alkenyl group, a C2-C22 alkynyl group, and a 5-10 membered aromatic hetero- or non-hetero mono- or bicyclic group,
wherein the C1-C22 alkyl group, the C2-C22 alkenyl group, and the C2-C22 alkynyl group is optionally substituted with 1 to 5 substituents independently selected from =O, -OH, -COOR₁, -F, -Cl, Br, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O,-OH, -COOR₁, -F, -Cl, Br, -C₁₋₆-alkyl, -C₂₋₆-alkenyl, -O-C₁₋₆-alkyl, -O-C₂₋₆-alkenyl, -CN, -CF₃,-SH and -NR₁R₂,
wherein the 5-10 membered aromatic group is optionally substituted with 1 to 5 substituents independently selected from =O, -OH, -COOR₁, -F, -Cl, Br, -C₁₋₆-alkyl, -C₂₋₆-alkenyl, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O, -OH, -COOR₁, -F, -Cl, Br, -C₁₋₆-alkyl, -C₂₋₆-alkenyl, -O-C₁₋₆-alkyl, -O-C₂₋₆-alkenyl, -CN, -CF₃, -SH and -NR₁R₂,
wherein R₁ and R₂ in the above definitions are independently selected from H and C₁-₆alkyl;
and with the proviso that at least one of D₁ and D₂ is a 5-10 membered aromatic group substituted with one or more -OH groups and/or one or more -COOH groups;
L₁ and L₂ represent the bonds by which D₁ and D₂ are connected to the tetrahydrofuran rings, with L₁ and L₂ being independently selected from a direct single or direct double bond, an ester bond, an amide bond, an ether bond, a ketoester bond (i.e. α-ketoester, β-ketoester), a carbamate bond, a carbonate bond, a carbamide bond, a phosphate ester bond, a phosphate amine bond, a phosphonic ester bond, a phosphite ester bond, a phosphonate bond, a phosphinite bond, a phosphorane thiocarbonate bond, a dithiocarbonate bond, a thioether bond, a sulphite bond, a sulfonate bond, a sulfanate bond, a sulfonate bond, a silanol bond, a silyl ether bond, a hydroxylamine bond, an oxime bond, an imine bond, a boronate ester bond and an imino ether bond.

It should be understood that the invention extends to all possible stereoisomers of the compounds according to Formula (1). Thus, any disclosure herein with respect to Formula (1) should also be taken as an explicit disclosure with respect to each of the configurations depicted in Formulae (1a), (1b), and (1c):

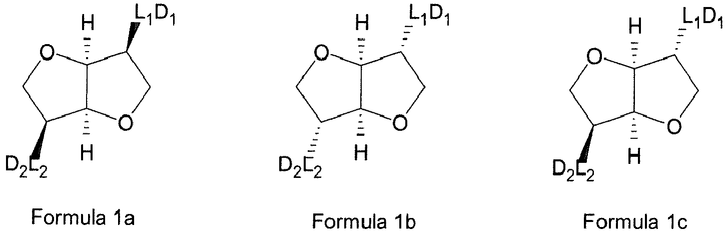

It is to be understood that any disclosure herein with respect to the compounds of Formula (1) also extends to tautomers, pharmaceutically acceptable salts, polymorphs, and solvates thereof. "Pharmaceutically acceptable salts", as used herein, are salts that retain the desired biological activity of the parent compound and do not impart undesired toxicological effects. Pharmaceutically acceptable salt forms include various crystalline polymorphs as well as the amorphous form of the different salts. The pharmaceutically acceptable salts can be formed with metal or organic counterions and include, but are not limited to, alkali metal salts such as sodium or potassium; alkaline earth metal salts such as magnesium or calcium; and ammonium or tetraalkyl ammonium salts. The salts can be organic or inorganic in nature. Representative salts include acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. "Solvates", as used herein, are addition complexes in which the compound is combined with an acceptable co-solvent in some fixed proportion. Co-solvents include, but are not limited to, ethyl acetate, lauryl lactate, myristyl lactate, cetyl lactate, isopropyl myristate, methanol, ethanol, 1-propanol, isopropanol, 1-butanol, isobutanol, tert-butanol, acetone, methyl ethyl ketone, acetonitrile, benzene, toluene, xylene(s), ethylene glycol, dichloromethane, 1,2-dichloroethane, *N*-methylformamide, *N,N-*dimethylformamide, *N*-methylacetamide, pyridine, dioxane, and diethyl ether. The term 'hydrate' is employed when the co-solvent is water.

For the purposes of the present invention at least one of D₁ and D₂ of Formula (1) - and hence also of Formula (1a), (1b) or (1c) - is a 5-10 membered aromatic group substituted with one or more -OH groups and/or one or more -COOH groups. D₁ and D₂ may be the same or different. That is D₁ and D₂ may be the same in that they have the same 5-10 membered aromatic group core structure with the same substituent pattern, or they may be different in that they either have the same 5-10 membered aromatic group core structure but different substituent pattern or have a different 5-10 membered aromatic group core structure altogether. In another embodiment of the invention, one of D₁ or D₂ is not a 5-10 membered aromatic group but instead is a different group altogether as will be explained in more detail later.

In a preferred embodiment, D₁ and/or D₂ is a 5-10 membered aromatic group substituted with two, three or four -OH substituents. In a preferred embodiment, D₁ and/or D₂ is a 5-10 membered aromatic group substituted with two, three or four -OH substituents and no -COOH substituents. In a preferred embodiment, D₁ and/or D₂ is a 5-10 membered aromatic group substituted with two, three or four -COOH substituents. In a preferred embodiment, D₁ and/or D₂ is a 5-10 membered aromatic group substituted with two, three or four -COOH substituents and no -OH substituents. In one embodiment, D₁ and/or D₂ is a 5-10 membered aromatic group substituted with one -OH substituent and one -COOH substituent; or with two -OH substituents and one -COOH substituent; or with three -OH substituents and one -COOH substituent; or with one -OH substituent and two -COOH substituents; or with two -OH substituents and two-COOH substituents; or with one -OH substituent and three -COOH substituents.

One way to obtain the desired -OH and -COOH substituent pattern is to prepare the compound according to Formula (1) from an aromatic hydroxy acid. Suitable aromatic hydroxy acids include, for example, a trihydroxybenzoic acid such as gallic acid (3,4,5-trihydroxybenzoic acid) and phloroglucinol carboxylic acid (2,4,6-trihydroxybenzoic acid) and derivatives thereof; a dihydroxybenzoic acid such as 2-pyrocatechuic acid (2,3-dihydroxybenzoic acid), β-resorcin acid (2,4-dihydroxybenzoic acid), gentisic acid (2,5-dihydroxybenzoic acid), γ-resorcin acid (2,6-dihydroxybenzoic acid), protocatechuic acid (3,4-dihydroxybenzoic acid), α-resorcin acid (3,5-dihydroxybenzoic acid), 3,6-dihydroxybenzoic acid and orsellinic acid, and derivatives thereof; a monohydroxybenzoic acid such as salicylic acid (2-hydroxybenzoic acid), 3-hydroxybenzoic acid, 4-hydroxybenzoic acid, creosotic acid, vanillic acid and syringic acid, and derivatives thereof; a phenylacetic acid such as mandelic acid, benzyl acid and atrolactic acid, and derivatives thereof; a hydroxycinnamic acid such as melilotic acid, phloretic acid, coumaric acid, umbellic acid, caffeic acid, ferulic acid and sinapic acid, and derivatives thereof; and an aromatic dicarboxylic acid having at least one hydroxy group such as 5-hydroxyisophthalic acid, 2-hydroxyterephthalic acid and 2,5-dihydroxyterephthalic acid. The fused tetrahydrofuran core structure having a desired stereochemistry can be provided, for example, by reaction with isosorbide, isomannide or isoidide.

In the present invention, the 5-10 membered aromatic group is optionally substituted with 1 to 5 substituents independently selected from =O, -OH, -COOR₁, -F, -Cl, Br, -C₁₋₆-alkyl, -C₂₋₆-alkenyl, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O, -OH, -COOR₁, -F, -Cl, Br, -C₁₋₆-alkyl, -C₂₋₆-alkenyl, -O-C₁₋₆-alkyl, -O-C₂₋₆-alkenyl, -CN, -CF₃, -SH and -NR₁R₂, and wherein R₁ and R₂ in the above definitions are independently selected from H and C₁₋₆-alkyl. Apart from the substituents -C₁₋₆-alkyl, -C₂₋₆-alkenyl, -O-C₁₋₆-alkyl, -O-C₂₋₆-alkenyl, and phenyl, all other substituents on the 5-10 membered aromatic group or phenyl substituent are terminal substituents. That is, they do not form part of bonds L₁ or L₂. Conversely, the atoms that are used for the bonds L₁ and L₂ are not counted as =O, -OH, -COOR₁, -F, -Cl, Br, -CN, -CF₃, -SH, or - NR₁R₂ substituents of the 5-10 membered aromatic group or phenyl substituent. In the case of the substituents -C₁₋₆-alkyl, -C₂₋₆-alkenyl, -O-C₁₋₆-alkyl, -O-C₂₋₆-alkenyl, and phenyl, these can be terminal groups or they can form part of the bonds L₁ and L₂. When they form part of the bonds L₁ and L₂ they have the function of acting as a spacer group between the fused tetrahydrofuran rings and the 5-10 membered aromatic group. By way of example, isosorbide digallate ester is considered to have three -OH substituents on each phenyl ring for the purposes of this invention. The -COOH group that would otherwise be present in gallic acid is not counted as a substituent since it is being used to provide the ester bond with the fused tetrahydrofuran rings.

Where the ring size permits, in addition to the various combinations of -OH and -COOH substituents described immediately above, the 5-10 membered aromatic group may be further substituted with one or more of =O, -COOR₁ (R₁ is C₁₋₆-alkyl), -F, -Cl, Br, -C₁₋₆-alkyl, -O-C₁₋₆-alkyl, -CN, -CF₃, -SH, -NR₁R₂, and phenyl, wherein said phenyl is optionally substituted with 1 to 3 substituents selected from =O, -OH, -COOR₁, -F, -Cl, Br, -C₁₋₆-alkyl, -O-C₁₋₆-alkyl, -CN, -CF₃, -SH and -NR₁R₂ wherein R₁ and R₂ are independently selected from H and C₁₋₆-alkyl unless stated otherwise. Alternatively, in addition to the various combinations of -OH and -COOH substituents described immediately above, the 5-10 membered aromatic group may contain no further substituents.

The 5-10 membered aromatic group is not particularly limited and may include a hydrocarbon 5-10 membered aromatic mono- or bi-cyclic system such as benzene or naphthalene, or it may be a mono- or bi-cyclic heterocyclic system containing up to four heteroatoms selected from N, O and S, such as pyrrole, furan, thiophene, imidazole, oxazole, isoxazole, thiazole, isothiazole, pyrazole, pyridine, pyridone, pyrazine, pyridazine, pyrimidine, pyrimidone, indazole, benzofuran, isobenzofuran, indole, isoindole, benzothiophene, benzo[c]thiophene, benzimidazole, purine, indazole, benzoxazole, benzisoxazole, benzothiazole, quinolone, quinoxaline, quinazoline, cinnoline and the like. It is preferred that the 5-10 membered aromatic group is a five- or six-membered group, and of these it is most preferred that benzene and pyridine being most preferred. It is to be understood that, structural restraints and chemistry permitting, this invention also extends to each of the preferred ring structures disclosed in this paragraph with each of the preferred combinations of -OH and -COOH substituents described in the preceding paragraphs. By way of example, the invention extends to embodiments where D₁ and/or D₂ is a benzene ring having the preferred combinations of -OH and -COOH substituents described in the preceding paragraphs. By way of a further example, the invention extends to embodiments where D₁ and/or D₂ is a pyridine ring having the preferred combinations of -OH and -COOH substituents described in the preceding paragraphs.

In a preferred embodiment, the invention relates to compounds of Formula (1) where D₁ and D₂ are the same and are a benzene ring with two or three -OH substituents. In another preferred embodiment, the invention relates to compounds of Formula (1) where D₁ and D₂ are different, with one of D₁ and D₂ being a benzene ring with two or three -OH substituents and the other being a benzene ring with one -Cl substituent at the ortho-, meta- or para-position to the linking group L₁ or L₂.

In the compounds of Formula (1) - and hence also of Formula (1a), (1b) and (1c) - the D₁ and D₂ groups are connected to the fused tetrahydrofuran rings via L₁ and L₂, respectively. L₁ and L₂ are independently selected from a direct single or direct double bond, an ester bond, an amide bond, an ether bond, a ketoester bond (i.e. α-ketoester, β-ketoester), a carbamate bond, a carbonate bond, a carbamide bond, a phosphate ester bond, a phosphate amine bond, a phosphonic ester bond, a phosphite ester bond, a phosphonate bond, a phosphinite bond, a phosphorane thiocarbonate bond, a dithiocarbonate bond, a thioether bond, a sulphite bond, a sulfonate bond, a sulfanate bond, a sulfonate bond, a silanol bond, a silyl ether bond, a hydroxylamine bond, an oxime bond, an imine bond, a boronate ester bond and an imino ether bond. To provide some examples, what is meant by an ether bond is that L₁ or L₂ is -O-; what is meant by an ester bond is that L₁ or L₂ is -C(=O)O-; what is meant by a carbonate bond is that L₁ or L₂ is -O-C(=O)-O-; what is meant by an amide bond is that L₁ or L₂ is -C(=O)N(R')-; what is meant by an amide bond is that L₁ or L₂ is -C(=O)N(R')-; what is meant by a carbamate bond is that L₁ or L₂ is-C(=O)ON(R')-; and so on wherein in each case R' is H or a C₁₋₆-alkyl group. In the present invention the L₁ or L₂ bond may be physiologically labile or physiologically stable. Preferred bonds are a direct single bond, an ester bond and an amide bond.

In the present invention, one of D₁ or D₂ may be a -H, -OH, -COOR₁, -F, -Cl, Br, -O-C₁₋₆-alkyl, -CN, -CF₃, -SH, -NR₁R₂, a C1-C22 alkyl group, a C2-C22 alkenyl group, a C2-C22 alkynyl group, wherein the C1-C22 alkyl group, the C2-C22 alkenyl group, and the C2-C22 alkynyl group is optionally substituted with 1 to 5 substituents independently selected from =O, -OH, -COOR₁, -F, -Cl, Br, -O-C₁₋₆-alkyl, -CN, -CF₃, -SH, -NR₁R₂, and phenyl, wherein said phenyl is optionally substituted with 1 to 3 substituents selected from =O, -OH, -COOR₁, -F, -Cl, Br, -C₁₋₆-alkyl, -O-C₁₋₆-alkyl, -CN, -CF₃, -SH and -NR₁R₂, and wherein R₁ and R₂ in the above definitions are independently selected from H and C₁₋₆-alkyl. For the purpose of treating a bacteria biofilm or potentiating an antibiotic, it is sufficient for the present invention that only one of D₁ or D₂ is a 5-10 membered aromatic group substituted with one or more -OH groups and/or one or more -COOH groups. The other of D₁ or D₂ can be modified to tailor the compound's hydrophobicity for a particular delivery system or use. By way of example, through a judicious choice of substituent, the compound according to Formula (1) can be made more hydrophilic if it is to be used in an aqueous detergent system or it can be made more hydrophobic if it is to be used in an oil-rich system such as an ointment.

Exemplary alkyl groups for the C1-C22 alkyl include methyl, ethyl, propyl, isopropyl, butyl, isobutyl, sec-butyl, tert-butyl, hexyl, heptyl, octyl, nonyl, decyl, undecyl, dodecyl, tridecyl, as well as alkyl groups derived from fatty acids such valeric acid, isovaleric acid, caproic acid, enanthic acid, caprylic acid, pelargonic acid, capric acid, undecylic acid, lauric acid, tridecylic acid, myristic acid, pentadecylic acid, palmitic acid, margaric acid, stearic acid, nonadecylic acid, and arachidic acid. Exemplary alkenyl groups for the C2-C22 alkenyl include ethylene, propylene, butylene, pentylene, hexylene, and octylene, including those derived from fatty acids such as myristoleic acid, palmitoleic acid, sapienic acid, oleic acid, elaidic acid, vaccenic acid, linoleic acid, linoelaidic acid, α-linolenic acid, and arachidonic acid.

Where one of D₁ or D₂ is not a 5-10 membered aromatic group then it is preferred that that D₁ or D₂ is selected from hydrogen, -OH, or is a hydrocarbon group selected from alkyl, alkenyl, alkylcarbonyl, and alkenylcarbonyl, wherein the alkyl group or moiety consists of unsubstituted or substituted, straight-chain or branched-chain or cyclic alkyl groups having 1-22 carbon atoms, and wherein the alkenyl moiety consists of unsubstituted or substituted, straight-chain or branched-chain or cyclic alkenyl groups having 2-22 carbon atoms. In this case, preferred combinations for L₁D₁ or L₂D₂ are -H, -OH or -R₃ (in which case L₁ or L₂ is a direct bond), or L₁D₁ or L₂D₂ is -COOR₃ (in which case L₁ or L₂ is an ester bond), wherein R₃ is a C1 to C22 hydrocarbon group. More preferred are those compounds where R₃ is a linear C1-C18 alkyl group, a branched C3-C18 alkyl group, a linear C2-C18 alkenyl group, or a branched C3-C18 alkenyl group. Preferred embodiments of this aspect of the invention are compounds where one of L₁D₁ or L₂D₂ is -H, -CH₃, -C₂H₅, -COCH₃, or -COC₂H₅.

Exemplary compounds for the present invention include:

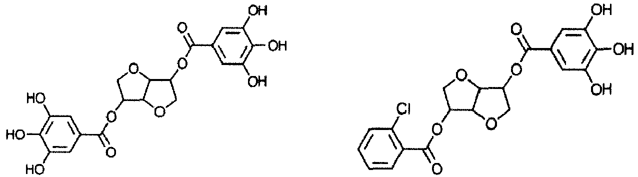

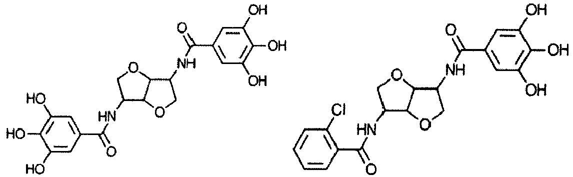

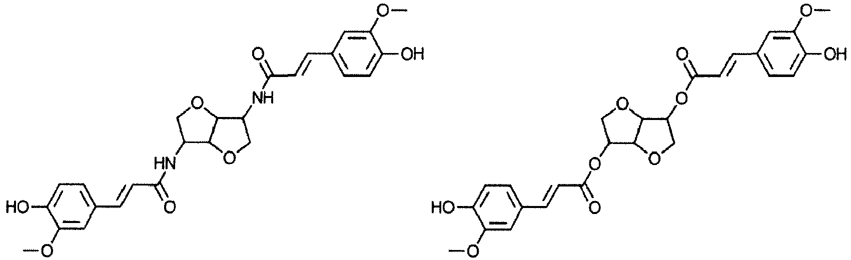

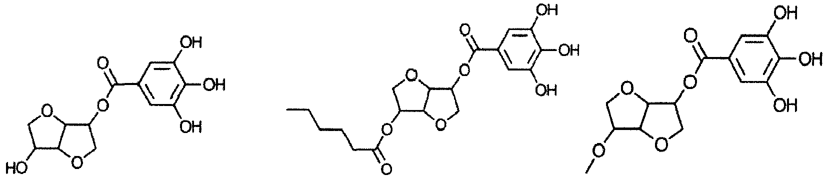

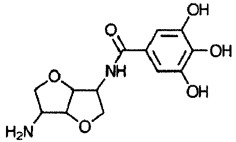

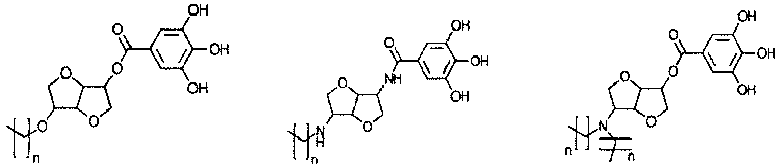

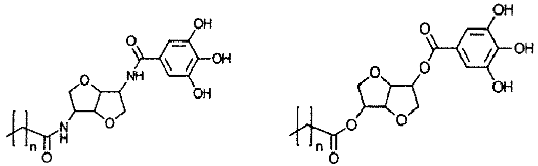

In the above formulae 'n' is any integer from 0 to 21, preferably 0, 1, 2, 3, 4, 5, 6, 7, 8, 9, 10, 11, or 12. Where there are two 'n's then each is independently any integer from 0 to 21.

### 5.3 Preparation of the compound according to Formula (1)

The compounds of the invention may be synthesized by any manner known in the art for linking two molecules together. By way of example, D₁/D₂ is condensed with e.g. a dianhydrohexitol moiety under conditions suitable for forming a linkage. In some cases it is necessary to block and/or protect some reactive groups on one, the other, or both of the moieties. Where the moieties are to be covalently linked via a linker, it is advantageous to first condense one of D₁, D₂ or the dianhydrohexitol moiety with the linker. In some cases it is advantageous to perform the reaction in a suitable solvent, such as acetonitrile, in the presence of suitable catalysts, such as carbodiimides including EDCI (1-ethyl-3-(3-dimethylaminopropyl)-carbodiimide) and DCC (DCC: dicyclohexylcarbodiimide), or under conditions suitable to drive off water of condensation or other reaction products (e.g., reflux), or a combination of two or more thereof. After the first moiety is condensed with the linker, the combined first moiety and linker may then be condensed with the remaining desired moieties. Again, in some cases it is advantageous to perform the reaction in a suitable solvent, such as acetonitrile, in the presence of suitable catalysts, such as carbodiimides including EDCI and DCC, or under conditions suitable to drive off water of condensation or other reaction products (e.g., reflux), or a combination of two or more thereof. Where one or more active groups have been blocked, it may be advantageous to remove the blocking and/or protecting groups under selective conditions, however it may also be advantageous, where the hydrolysis product of the blocking and/or protecting group and the blocked group is physiologically benign, to leave the active groups blocked. Such the blocking and/or protecting are well known in the art and the interest reader can refer to W. Green, P. G. M. Wuts, Protective Groups in Organic Syntheses, Wiley-Interscience, New York, 1999**.**

Exemplary reaction schemes according to the present invention are illustrated below for linking D₁/D₂ with a dianhydrohexitol (Z). These schemes can be generalized by substituting various groups having at least one functional group that can form a covalent bond to another compound having a similar or different functional group, either directly or indirectly through the desired linker. The person of skill in the art will appreciate that these schemes also may be generalized by using other appropriate linkers.

### Schema 1 (ester linkage) D₁-COOH + HO-Z → D₁-COO-Z

In this case D₁ is joined to the dianhydrohexitol by an ester linkage. Referring to Formula (1), the linking group L₁ in this case would be -C(=O)O- (i.e. an ester bond).

Dianhydrohexitol esters are usually obtained by direct esterification of the dianhydrohexitol with the corresponding carboxylic acid in presence of a catalyst (US 8,258,325 B2, US 2012/0116101 A1; US 6,693,209 B2; WO 99/45060). Transesterification of dianhydrohexitol with the ester have also been reported (US 8,258,325 B2). Esterification and transesterification can also be used to generate mono substituted dianhydrohexitol (US 2,983,228) as well as reaction starting from acid anhydride (Lavergne et al. RSC Adv. 2013, 3, 5997). Diester and monoester derivatives can also been obtained by reaction of dianhydrohexitol with acid halide (Machut et al. Green Chem. 2010, 12, 772; Lavergne et al. RSC Adv. 2013, 3, 5997), and isosorbide diaspirinate can be obtained by this synthetic route (Gilmer et al. Eur. J. Phar. Sci. 2002 16, 297). Sensitive carboxylic acid substrate that would degrade when subject to the reaction condition used in the protocols mentioned above can be attached to isosorbide under milder condition using, for instance carbodiimide coupling chemistry. (Gella et al. Mol. Cryst. Liq. Cryst. 2014 591, 34; EP 2 332 942 A1).

### Schema 2 (carbonate linkage) D₁-O-CO-X + HO-Z → D₁-O-CO-O-Z

Dianhydrohexitol carbonate derivatives can be synthesized by reaction with haloformate (Kircheldorf et al. Macromol. 1996, 29, 8077; Saito et al. Macromol. 2005, 38, 6485; Yokoe et al. J. Pol. Sci. A 2003, 41, 2312).

### Scheme 3 (ether linkage) D₁-X + HO-Z → D₁-O-Z

Dianhydrohexitol ether derivatives can be synthesized via Williamson reaction (Achet et al., Biomass 1986, 9, 247; Chatti et al. Tetrahedron 2000, 56, 5877; Zhu et al Green Chem., 2008, 10, 532; Zhu, et al., Langmuir 2009, 25, 13419.

### Scheme 4 (carbamate linkage) D₁-NCO + HO-Z → D₁-N-CO-O-Z

Dianhydrohexitol carbamate derivatives can also be obtained by reaction with isocyanate (Dillon et al, Bioorg. Med. Chem. 2010, 18, 1045).

### Scheme 5 (amide linkage) D₁-COOH + H₂N-Z → D₁-CO-NH-Z

Amides derivatives can be synthesized by reaction with carboxylic acid with a primary amine (WO2011/002871) or by reacting acyl halide with a primary amine (Vermote et al. Angew. Chem. Int. Ed. 2016, 55, 6551-6555; WO 2016/005340 A1).

### 5.4 Treatment of bacterial infections and biofilms

The compounds having Formula (1) are suitable for use in the treatment of biofilm and the prevention of its formation. In one embodiment of the invention, the compounds having Formula (1) are used to reduce the effective dose of antibiotic required to eliminate a biofilm. In another embodiment, the compounds having Formula (1) are used to increase efficacy of an antibiotic without increase its dosage. In another embodiment, the compounds having Formula (1) are used to prevent a biofilm from forming.

The term *"biofilm"* is to be understood as having its ordinary meaning. A biofilm is a sessile community of microorganisms characterized by cells that are attached to a substratum or interface or to each other, that are embedded in a matrix of extracellular polymers (more specifically extracellular polymers that they have produced), and that exhibit an altered phenotype with respect to growth rate and gene transcription (for example as compared to their "non-biofilm" or free-floating or planktonic counterparts).

The biofilms that may be treated in accordance with the present invention are not limited in terms of the microorganisms that they contain. The biofilm may comprise any class, genus or species of microorganism, namely any microorganism that may form a biofilm. Such microorganisms typically include bacteria, including any genus or species of bacteria. Thus, the bacteria may be gram positive or gram negative, or gram test non-responsive.

The term 'Gram-positive bacteria' is an art recognized term for bacteria characterized by having as part of their cell wall structure peptidoglycan as well as polysaccharides and/or teichoic acids and are characterized by their blue-violet color reaction in the Gram-staining procedure. Representative Gram-positive bacteria include: *Actinomyces* spp., *Bacillus anthracis, Bifidobacterium* spp., *Clostridium botulinum, Clostridium perfringens, Clostridium* spp., *Clostridium tetani, Corynebacterium diphtheria, Corynebacterium jeikeium, Enterococcus faecalis, Enterococcus faecium, Erysipelothrix rhusiopathiae, Eubacterium* spp., *Gardnerella vaginalis, Gemella morbillorum, Leuconostoc* spp., *Mycobacterium abcessus, Mycobacterium avium complex, Mycobacterium chelonae, Mycobacterium fortuitum, Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia* spp., *Peptococcus niger, Peptostreptococcus* spp., *Proprionibacterium* spp., *Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae* (group B *streptococcus), Streptococcus anginosus, Streptococcus bovis, Streptococcus canis, Streptococcus equi, Streptococcus milleri, Streptococcus mitior, Streptococcus mutans, Streptococcus pneumoniae, Streptococcus pyogenes* (group A *streptococcus), Streptococcus salivarius,* and *Streptococcus sanguis.*

The term "Gram-negative bacteria" is an art recognized term for bacteria characterized by the presence of a double membrane surrounding each bacterial cell. Representative Gram-negative bacteria include *Acinetobacter calcoaceticus, Actinobacillus actinomycetemcomitans, Aeromonas hydrophila, Alcaligenes xylosoxidans, Bacteroides, Bacteroides fragilis, Bartonella bacilliformis, Bordetella* spp., *Borrelia burgdorferi, Branhamella catarrhalis, Brucella* spp., *Campylobacter* spp., *Chlamydia pneumoniae, Chlamydia psittaci, Chlamydia trachomatis, Chromobacterium violaceum, Citrobacter* spp., *Eikenella corrodens, Enterobacter aerogenes, Escherichia coli, Flavobacterium meningosepticum, Fusobacterium* spp., *Haemophilus influenzae, Haemophilus* spp., *Helicobacter pylori, Klebsiella* spp., *Legionella* spp., *Leptospira* spp., *Moraxella catarrhalis, Morganella morganii, Mycoplasma pneumoniae, Neisseria gonorrhoeae, Neisseria meningitidis, Pasteurella multocida, Plesiomonas shigelloides, Prevotella* spp., *Proteus* spp., *Providencia rettgeri, Pseudomonas aeruginosa, Pseudomonas* spp., *Rickettsia prowazekii, Rickettsia rickettsii, Rochalimaea* spp., *Salmonella* spp., *Salmonella typhi, Serratia marcescens, Shigella* spp., *Treponema carateum, Treponema pallidum, Treponema pallidum endemicum, Treponema pertenue, Veillonella* spp., *Vibrio cholerae, Vibrio vulnificus, Yersinia enterocolitica, Yersinia pestis.*

The bacteria may be aerobic or anaerobic. The bacteria may be pathogenic or non-pathogenic. It is particularly surprising that the compounds according to Formula (1) are able to disrupt the formation of a biofilm and improve the efficacy of an antibiotic to kill bacteria in biofilms. In one embodiments, biofilms comprising or consisting of *Pseudomonas aeruginosa,* or *Pseudomonas spp* are targets for the present invention. In one embodiment, the compound according to Formula (1) is isosorbide digallate ester and the biofilm to be treated or prevented is one comprising *Pseudomonas aeruginosa*. Biofilms may also contain fungi, algae and other organisms such as parasitic protozoa in addition to the bacteria. Such mixed colony biofilms are also treatable according to the methods described herein.

In one embodiment of the invention the compounds according to Formula (1), optionally together with an antibiotic, are used to treat a biofilm-associated infection. A *"biofilm-associated infection"* is a microbial infection of an object or a subject where it is known or suspected that the microbes are present as a biofilm. As used herein, a subject refers to a human or any non-human animal subject. Typically a biofilm-associated infection will be an infection where the existence of a biofilm is relevant to a particular clinical condition, e.g. to the diagnosis or prognosis, to the treatment regimen, to the severity of the infection, to the duration of the infection up to the point of treatment or anticipated in the future. Exemplary infections to be treated with compounds according to the present invention include microbial infections in the body such as urinary tract infections, catheter associated and middle-ear infections, formation of dental plaque, gingivitis, coating contact lenses, and less common but more lethal processes i.e. endocarditis, infections in cystic fibrosis, and infections of permanent indwelling devices such as joint prostheses and heart valves.

The present invention is suited to treating biofilms in chronic wounds. These include, but are not limited to, venous ulcers, diabetic ulcers, and pressure ulcers. Venous ulcers, for example, occur mostly in the legs, as a result of poor circulation (*e.g*., ischemia), malfunctioning valves of veins, or repeated physical trauma (*e.g*., repetitive injury). Pressure ulcers may be present when local pressure that is exerted at or around a wound site is greater than blood pressure, for instance, such that poor circulation, paralysis, and/or bed sores may contribute to, or exacerbate, the chronic wound. Diabetic ulcers may occur in individuals with diabetes mellitus, for example, persons in whom uncontrolled high blood sugar can contribute to a loss of feeling in the extremities, leading to repetitive injuries and/or neglect on the part of the individual to attend to injuries. Biofilms in chronic wounds that have resulted from acute wounds such as gunshot or shrapnel wounds, burns, punctures, or surgical wounds (*e.g*. postoperative wound infections) can also be targeted by compounds according to Formula (1). Biofilms can be treated in other wounds such as those arising from radiation poisoning, malignancies, dermal infections, gangrene, non-healing surgical wounds, pyoderma gangrenosum, traumatic wounds, acute arterial insufficiency, necrotizing fasciitis, and osteomyelitis (bone infection).

*"Treatment"* of a biofilm includes prophylactic treatment and encompasses a reduction in size of the biofilm, a reduction in number of living microorganisms within the biofilm and prevention or reduction in the tendency of microorganisms within the biofilm to break free and form new biofilm colonies. The size, structure, integrity, and number of microbes in a biofilm can be analysed by any convenient method. For instance, scanning and transmission electronic microscopy is often used to assess the size, integrity and structure of a biofilm. When a subject is treated, treatment includes an improvement, observed by clinician or patient, in one or more of the symptoms associated with the infection.

The biofilm to be treated may be present on a surface. The surface is not limited and includes any surface on which a microorganism may occur. The surface may be biotic or abiotic, and inanimate (or abiotic) surfaces include any such surface which may be exposed to microbial contact or contamination. Thus particularly included are surfaces exposed to microbial contact or contamination include in particular any part of: food or drink processing, preparation, storage or dispensing machinery or equipment, air conditioning apparatus, industrial machinery, e.g. in chemical or biotechnological processing plants, storage tanks and medical or surgical equipment. Examples of bacterial outbreaks attributed to environmental contamination include tuna salad (C. jejuni), ice cream (S. enteritidis), infant formula (S. ealing), soft cheese (S. berta), cooked sliced ham (S. typhimurium), chocolate (S. Napoli; S. eastbourne), butter (L. monocytogenes), hot dogs (L. monocytogenes), canned salmon (C. botulinum), lasagna (S. aureus), various different foods (E. coli), chocolate milk (Y. enterocolitica), canned meat (S. typhi), crabmeat (S. aureus), canned mushrooms (S. aureus), pastry (S. Enteritidis), yeasts (S. München), Pasteurized milk (S. typhimurium; E. coli; B. cereus; Y. enterocolitica). The compounds according to Formula (1) can be used to treat industrial and home surfaces to prevent such outbreaks. The compounds of formula (1) can also be used to prevent contamination of food processing equipment that comes into contact with bacteria that are otherwise beneficial in food products such as Lactobacillus spp, Acetobacter aceti, Streptococcus thermophilus and Lactobacillus bulgaricus.

A biotic or animate surface may include any surface or interface in or on the human or animal body. It may be any internal or external body surface, including of any tissue, which may include haematological or haemotopoietic tissue (e.g. blood). Dead or dying (e.g. necrotic) or damaged (e.g. inflamed or disrupted or broken) tissue is particularly susceptible to biofilm growth and such tissue is encompassed by the term "animate" or "biotic". The surface may be a mucosal or non-mucosal surface. Representative biotic surfaces include, but are not limited to any surface in the oral cavity, e.g. teeth, gingiva, gingival crevice, periodontal pocket, reproductive tract (e.g. cervix, uterus, fallopian tubes), the peritoneum, middle ear, prostate, urinary tract, vascular intima, conjunctiva, corneal tissue, the respiratory tract, lung tissue (e.g. bronchial and alveolar), heart valves, gastrointestinal tract, skin, scalp, nails and the interior of wounds, particularly chronic wounds, which may be topical or internal wounds.

Medical or surgical equipment or devices represent a particular class of surface on which a biofilm may form. This may include any kind of line, including catheters (e.g. central venous and urinary catheters), prosthetic devices *e.g*., heart valves, vascular grafts, artificial joints, false teeth, dental crowns, dental caps and soft tissue implants (*e.g*. breast, buttock and lip implants). Any kind of implantable (or "in-dwelling") medical device is included (*e.g*. stents, intrauterine devices, pacemakers, intubation tubes, prostheses or prosthetic devices, lines or catheters). An "in-dwelling" medical device may include a device in which any part of it is contained within the body, i.e. the device may be wholly or partly in-dwelling. Medical or surgical equipment on which a biofilm has already formed can be treated in accordance with the invention. To prevent or hinder the formation of a biofilm in the first place, the medical or surgical equipment can comprise a compound according to Formula (1), for instance, on a part or the whole of a surface thereof. The present invention therefore also relates to medical devices comprising a compound according to Formula (1). In a preferred embodiment of the invention, the medical device is selected from the group consisting of grafts, membranes, tubes, connectors, surgical instruments, intra-aortic balloons, stents (including drug-eluting vascular stents), blood bags, catheters, sutures, prostheses, heart valves, tissue adhesives, cardiac pacemaker leads, artificial organs, lenses for the eye, blood handling equipment, apheresis equipment, (bio)sensors, dental devices, skin patches, wound dressings (including drug-eluting wound dressings), implantable devices, tampons, bandages, drug delivery systems, bodily implants, and protein-eluting scaffolds for tissue regeneration.

Wounds to be treated may be acute or chronic. Acute wounds are wounds that orderly progress through the three recognised stages of the healing processes (*i.e.* the inflammatory stage, the proliferative stage and the remodelling phase) without a protracted time course. Chronic wounds, however, are those wounds that do not complete the ordered sequence of biochemical events because the wound has stalled in one of the healing stages. Viewed alternatively a chronic wound is a wound that has not healed within at least 30 days, preferably at least 40 days, more preferably at least 50 days, most preferably at least 60 days. The wound to be treated may be a breach in, or denudement of, the tissue for instance caused by surgical incision or trauma, *e.g*., mechanical, thermal, electrical, chemical or radiation trauma; a spontaneously forming lesion such as a skin ulcer (*e.g.* a venous, diabetic or pressure ulcer); a blister (*e.g.* a friction or thermal blister or a blister caused by pathogen infection such as chicken pox); an anal fissure or a mouth ulcer.

### 5.5 Formulations

Whether the compounds according to Formulae (1) are to be used for treating biofilms or preventing its appearance, the compound can be used alone or as a formulation comprising the compound. For instance, when the compounds according to Formula (1) are used to treat a biotic surface, the compounds can be formulated as a composition suitable for topical administration. *"Topical administration"* in the sense of the present invention means administration to a definite surface, *e.g*. directly to the biofilm or wound surface or the area around the biofilm or wound surface. Suitable topical administration forms include lotions, creams, gels, ointments, pastes (e.g. toothpaste), emulsions, foams, mousses, solutions (e.g. mouthwash), sprays, dispersions, aerosols, alginates, hydrogels, hydrocolloids, sticks, bars, and films. Reference is made to *"*Remington: The Science and Practice of Pharmacy, 21st Ed., Chapter 39*"* and *"*Ansel's Pharmaceutical Dosage Forms and Drug Delivery Systems, 9th Ed., Section IV-10 and Section VI*"* for further information regarding topical formulations. Preferred topical formulations are lotions, creams, gels, and ointments. A suitable dosage of the formulation can be determined by the skilled person depending on the constituent drugs and according to the desired treatment. Where the compounds according to Formula (1) are used to treat an abiotic surface then any suitable formulation can be used (e.g. detergent solution, contact lens solution, denture-soaking solution, dissolvable pellets or tablets, coatings loaded with a compound according to Formula (1), and such like).

The topical formulations of the present invention typically contain 1 to 1,000,000 µM, preferably from 1000 to 100,000 µM of the compound according to Formula (1). Alternatively or additionally, the topical formulations of the invention contain from 0.0001 to 100 wt.%, preferably 0.0005 to 10 wt.%, more preferably 0.001 to 2 wt.%, even more preferably 0.01 to 0.1 wt.% of the compound according to Formula (1). If a mixture of two or more of the compounds of Formula (1) is present, the above ranges apply to the sum of the weight contents of those compounds. Exemplary, but non-limiting, dosages when applied to a surface (e.g. skin or wound surface) include an amount that allows effective administration of a compound of Formula (1) in an amount of 0.1 to 1,000,000 mg/m² or preferably 10 to 100,000 mg/m². The compounds of Formulae (1) can also be administered to a biofilm or wound by injection and thus can be formulated as an injection solution.

The compounds of Formula (1) can be used in simultaneous or sequential combination with one or more antibiotics. For example, when applied topically the compound can be applied before, together with, or after an antibiotic agent that is normally delivered onto or through a biotic surface for either a local or systemic effect. When applied simultaneously, the compound of the invention according to Formula (1) and the antibiotic agent may be present in the same topical formulation (e.g. in the form of a lotion, cream, gel, or ointment).

The antibiotic agent can be one or more antibiotic agents selected from iscapreomycins, such as capreomycin IA, capreomycin IB, capreomycin IIA and capreomycin IIB; carbomycins, such as carbomycin A; carumonam; cefaclor, cefadroxil, cefamandole, cefatrizine, cefazedone, cefazolin, cefbuperazone, cefcapene pivoxil, cefclidin, cefdinir, cefditoren, cefime, ceftamet, cefinenoxime, cefinetzole, cefminox, cefodizime, cefonicid, cefoperazone, ceforanide, cefotaxime, cefotetan, cefotiam, cefoxitin, cefpimizole, cefpiramide, cefpirome, cefprozil, cefroxadine, cefsulodin, ceftazidime, cefteram, ceftezole, ceftibuten, ceftiofur, ceftizoxime, ceftriaxone, cefuroxime, cefuzonam, cephalexin, cephalogycin, cephaloridine, cepholosporin C, cephalothin, cephapirin, cephamycins, such as cephamycin C, cephradine, chlortetracycline; chlarithromycin, clindamycin, clometocillin, clomocycline, cloxacillin, cyclacillin, danofloxacin, demeclocyclin, destomycin A, dicloxacillin, dicloxacillin, dirithromycin, doxycyclin, epicillin, erythromycin A, ethanbutol, fenbenicillin, flomoxef, florfenicol, floxacillin, flumequine, fortimicin A, fortimicin B, forfomycin, foraltadone, fusidic acid, gentamycin, glyconiazide, guamecycline, hetacillin, idarubicin, imipenem, isepamicin, josamycin, kanamycin, leumycins such as leumycin A1, lincomycin, lomefloxacin, loracarbef, lymecycline, meropenam, metampicillin, methacycline, methicillin, mezlocillin, micronaomicin, midecamycins such as midecamycin A1, mikamycin, minocycline, mitomycins such as mitomycin C, moxalactam, mupirocin, nafcillin, netilicin, norcardians such as norcardian A, oleandomycin, oxytetracycline, panipenam, pazufloxacin, penamecillin, penicillins such as penicillin G, penicillin N and penicillin O, penillic acid, pentylpenicillin, peplomycin, phenethicillin, pipacyclin, piperacilin, pirlimycin, pivampicillin, pivcefalexin, porfiromycin, propiallin, quinacillin, ribostamycin, rifabutin, rifamide, rifampin, rifamycin SV, rifapentine, rifaximin, ritipenem, rekitamycin, rolitetracycline, rosaramicin, roxithromycin, sancycline, sisomicin, sparfloxacin, spectinomycin, streptozocin, sulbenicillin, sultamicillin, talampicillin, teicoplanin, temocillin, tetracycline, thostrepton, tiamulin, ticarcillin, tigemonam, tilmicosin, tobramycin, tropospectromycin, trovafloxacin, tylosin, and vancomycin, as well as pharmaceutically acceptable salts, esters, prodrugs and protected forms thereof. The compounds of Formula (1) are particularly suited for use in combination with tetracycline.

Additionally, further active agents can be included in the composition if required. suitable additional active agent include analgesics, anesthetics, anti-inflammatory agents (e.g., steroidal compounds such as dexamethasone, betamethasone, prednisone, prednisolone, triamcinolone, hydrocortisone, alclometasone, amcinonide, diflorasone, etc. as well as non-steroidal antiinflammatories), anti-itch and irritation-reducing compounds (e.g., antihistamines such as diphenhydramine and psoriasis treatments), antimicrobial agents, antiseptic agents (e.g., povidone-iodine, methylbenzethonium chloride, etc.), immunomodulating agents, vitamins and the like.

The dosage (*i.e.* of the compound of formula (1) and/or the antibiotic is present) may be delivered by a single administration or by multiple applications as needed to achieve the most effective results. Dosing can continue for as long as is indicated, which will depend on the severity of the biofilm. The dosage may also be delivered in a controlled-release manner such as with an adhesive patch, suture, pellet, coating or gel.

For wounds created during surgery the compounds according to Formula (1) (or a formulation comprising these compounds) are preferably applied to the open wound and again on the closed wound on a regular basis for at least one week. An example of a regular basis is one, two or three separate applications per day. An alternative option is to apply the active agent by means of an adhesive patch or suture.

The present invention further relates to an adhesive patch comprising at least one compound according to Formula (1). Adhesive patches are a particularly effective means of delivering the compounds to a biofilm or wound. The adhesive patch of the invention comprises a backing layer and an adhesive layer disposed on at least one side of the backing. The adhesive patch of the invention can be sterilised by appropriate methods such as but not limited to irradiation. When selecting a suitable sterilization method, it is advisable to test and confirm that the sterilization does not lead to degradation or other inactivation of the compound of Formula (1).

The backing layer is preferably one which is substantially impermeable to components of the adhesive layer, such as a drug and additives, and prevents the components from passing through the backing and evaporating off from the back to cause a decrease in content. The backing layer may also provide odour control exudate control. Examples of such backings include films of polyurethanes, polyesters such as poly(ethylene terephthalate), nylons, Saran^{™}, polyethylene, polypropylene, poly(vinyl chloride), ethylene/ethyl acrylate copolymers, polytetrafluoroethylene, Surlyn^{™}, and metal foils and laminated films composed of two or more thereof. Moisture vapor permeability (MVTR) of the film is not particularly limited but may, if required, be adjusted to range from about 300 to 3000 g/m²/24h, preferably from about 500 to 2000 g/m²/24h, more preferably from about 700 to 1700 g/m²/24h as measured by the water method in ASTM E96.

The adhesive layer is not particularly limited and examples of suitable adhesives include those based on acrylic polymers; rubber-based adhesives such as styrene/diene/styrene block copolymers (*e.g*., styrene/isoprene/styrene block copolymers and styrene/butadiene/styrene block copolymers), polyisoprene, polyisobutylene, and polybutadiene; silicone type adhesives such as silicone rubbers, dimethylsiloxane-based polymers, and diphenylsiloxane-based polymers; vinyl ether type adhesives such as poly(vinyl methyl ether), poly(vinyl ethyl ether), and poly(vinyl isobutyl ether); vinyl ester type adhesives such as vinyl acetate/ethylene copolymers; and polyester type adhesives produced from a carboxylic acid ingredient such as dimethyl terephthalate, dimethyl isophthalate, or dimethyl phthalate and a polyhydric alcohol ingredient such as ethylene glycol. The adhesive layer may be either a cross-linked adhesive layer or an uncross-linked adhesive layer. Preferably, the adhesive layer is a pressure-sensitive adhesive layer. From the standpoint of adhesion to the skin, hydrophobic pressure-sensitive adhesives are preferred and pressure-sensitive adhesive layers containing no water are preferred.

A release liner may also be disposed on the pressure-sensitive adhesive layer to improve ease of handling such as, for example, protect the adhesive layer and provide additional support (e.g. stiffness) before the patch is applied to a wound. The release liner is usually discarded before the adhesive patch is applied to the skin. It is therefore preferred, from the standpoint of inhibiting the adhesive patch from giving an uncomfortable feeling during wear on the skin, that the backing should be softer than the release liner. The release liner is not particularly limited. Examples of the material thereof include materials which are known in this field. Suitable release liner materials may, for instance, include bases having a release layer of a plastic film or paper which surface is treated with a release agent such as a silicone, long-chain alkyl, fluorine, or molybdenum sulphide release agent; low-adhesive bases comprising fluorine-containing polymers; and low adhesive bases comprising non-polar polymers including olefinic resins such as polyethylenes and polypropylenes to improve release property from an adhesive layer. Specific examples thereof include films of plastics such as polyesters including poly(ethylene terephthalate), poly(vinyl chloride), poly(vinylidene chloride), various acrylic and methacrylic polymers, polystyrene, polycarbonates, polyimides, cellulose acetate (acetate), regenerated cellulose (cellophane), and celluloid and laminated films composed of wood-free paper, glassine paper, or the like and a polyolefin. From the standpoints of safety, profitability, and drug migration prevention, it is preferred to use a polyester film. A preferred option is a fluorinated siliconized material.

The thickness of the release liner is generally 10-200 µm, preferably 25-100 µm. It is preferred to use a release liner having maximum peel force of less than 0.5 N/25mm when measured at a 180° peeling angle.

Drug-containing adhesive patches are well known and examples of how to prepare them can be found, for example, in WO 2013/005760 A1, EP 2332585 A1, EP 2570122 A1, EP 2702984 A1, WO 2012/014589 A1, WO 2009/124763 A2, WO 98/21578 A1, and the further references cited therein. Compounds according to Formula (1) can be employed in the patch in the skin-contact adhesive or in one or more additional layers in the patch (e.g. in a drug reservoir). Suitable drug-delivery patches include gelled or liquid reservoirs, such as in US 4,834,979, so-called "reservoir" patches; patches containing matrix reservoirs attached to the skin by an adjacent adhesive layer, such as in US 6,004,578, so-called "matrix" patches; and patches containing PSA reservoirs, such as in US 6,365, 178, US 6,024,976, US 4,751, 087 and US 6,149,935, so-called "drug-in-adhesive" patches.

In some embodiments, the drug reservoir can be provided in the form of a matrix layer containing drug, the matrix layer being adhered to the skin-contact adhesive of the patch. Such a matrix can be an adhesive layer and can include any of the adhesives described above. Alternatively, the matrix layer can be non-adhesive or weakly adhesive and rely upon a surrounding rim of skin-contact adhesive to secure the patch in place and keep the drug reservoir in contact with the skin surface.

Another topical administration form according to the present invention is a dressing with an absorbing patch in the middle (standard plaster type) where the compound according to Formulae (1) is provided in the absorbing patch or on the surface in a resorbable matrix. In the first case the material will be into the absorbing material and be released by either exudate or skin moisture. If needed a secondary separating membrane could be used to ensure the correct water transport. In the last embodiment the compounds are embedded in a resorbable material such as but not limited to PLA, alginate, or CMC that will release the compound upon contact with moisture. This film can have several architectures such as but not limited to a film, a porous film, a mesh, a fabric but it should allow the proper liquid transport. Underneath this film another potential absorbing material can be layered in order to capture the liquid such as blood, exudate, or sweat. In some embodiments, the drug reservoir can be provided within the skin-contact adhesive of the patch. The drug can be mixed with the skin-contact adhesive prior to forming the patch or it may be applied to the skin-contact adhesive of the patch in a separate process step. Examples of suitable methods for applying drug to an adhesive layer may be found in US 2003/054025 and US 5,688,523.

Commercially available materials that can be modified to deliver compounds according to Formula (1) in the form of an adhesive patch include hydrocolloid dressing materials (e.g. DuoActive (Convatec), Comphile (Coloplast), Tegasorb (3M), Absocure^{™} (Nitto Medical Corporation), alginate dressing materials (e.g. Cartstat (Convatec), Sorbsan (Alcare Co., Ltd), Algodam (Medion Inc.), Kurabio AG (Kuraray Co., Ltd) ), a hydrogen dressing materials (e.g. Jelleypalm (Taketora), New Jail (Johnson & Johnson), Intrasite (Smith & Nephew), Granugel (Convatec), Clearsite (Nippon Sigmax Co., Ltd)), polyurethane dressing materials (e.g. Tegaderm (3M), Opsite Wound (Smith & Nephew), IV3000 (Smith & Nephew), Bioclusive (Johnson & Johnson)), hydropolymer dressing materials (e.g. Tieral (Johnson & Johnson)), and hydrofiber dressing materials.

By administering the compounds according to Formula (1) by means of an adhesive patch, one can ensure a more uniform application of the compound as compared to alternative topical formulations. Furthermore, the adhesive patch serves to protect the wound while it is healing. Preferably, the active compounds of the invention are formulated in the adhesive patch in an amount of 0.1 to 1,000,000 mg/m² or preferably 10 to 100,000 mg/m².

The present invention is now illustrated in greater detail by way of the following Examples, but it should be understood that the present invention is not deemed to be limited thereto.

### 6. Examples

### 6.1 Synthesis of a compound according to Formula (1)

Isosorbide digallate ester was synthesized in two steps from isosorbide and 3,4,5-tribenzyloxybenzoyl chloride as follow (3,4,5-tribenzyloxybenzoyl chloride was prepared as previously reported in Malik et al., Chemistry A European Journal 2012, 18(29), 9063-9074): 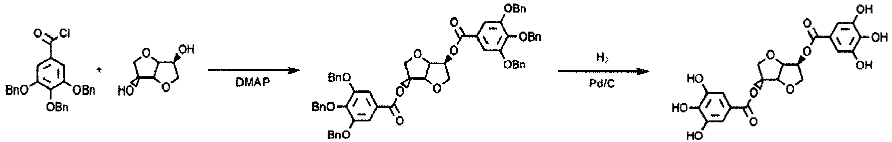 *Step 1*. In a nitrogen purged three necks flask fitted with a gas adaptor, a condenser and an addition funnel; 2.19 g isosorbide (15 mmol) and 4.59 g 4-dimethylaminopyridine (37.5 mmol - 2.5 eq.) were dissolved in 50 ml anhydrous toluene. A solution of 14.47 g 3,4,5-tribenzyloxybenzoyl chloride (31.5 mmol - 2.1 eq) in 50 ml anhydrous toluene was added dropwise to this solution under nitrogen atmosphere over 30 min. Once the addition completed, the cloudy mixture was heated and maintained at reflux for 5h. After cooling, the dark mixture was filtered, washed twice with 100 ml brine, dried over MgSO₄ and concentrated under reduced pressure leaving 12.4 g (83%) of the benzyl protected diester compound as a pale yellow solid. This product was used for next step without further purification.

*Step 2.* In a nitrogen purged Schlenk flask, 1.5 g of the benzyl protected diester compound (1.5 mmol) was dissolved in 45 ml of MeOH/DCM mixture (1 to 2 volume ratio). 0.15 g of Pd/C (10 wt%) was added and the mixture was stirred under H₂ atmosphere for 8h at room temperature. Next, the reaction mixture was filtered and the solvent was evaporated under reduced pressure leaving analytically pure isosorbide digallate ester.

### 6.2 Biofilm eradicating effect

For biofilm inhibition tests, the following saline-based samples were prepared

| Control | Saline solution |
|---|---|
| Sample A | 20 mM isosorbide digallate ester + 20 mM tetracycline |
| Sample B | 20 mM tetracycline |
| Sample C | 20 mM isosorbide + 40 mM gallic acid + 20 mM tetracycline |

All samples were tested in an *in vitro* model as described in Brackman et al., Journal of applied microbiology 2013, 114(6), 1833-1842 in order to determine the biofilm inhibitory effect of each samples. Briefly, *P. aeruginosa* grown overnight in solution were seeded in an artificial matrix and allowed to grow for 24h at 37°C to form a biofilm. The medium was then added with compounds at mentioned concentration and put back at 37°C for another 24 hr. After this the medium was removed, biofilms were washed with PS and then put into saline. After sonication and vortexing, suspensions were plated and the resulting CFUs/biofilms was determined. The results are shown in Table 1 and Figure 1.

**Table 1.**

| **Composition** | **CFU/AD** | **Log CFU/AD** |
|---|---|---|
| **Control (no treatment)** | 5.09 x 10⁹ | 9.71 |
| **Sample A** | < DL | < DL |
| **Sample B** | 2.34 x 10³ | 3.37 |
| **Sample C** | 1.02 x 10² | 2.01 |

| | | |
|---|---|---|
| *DL = detection limit | | |

Compositions A, B and C provided a marked biofilm eradicating effect. Surprisingly, Composition A comprising isosorbide digallate ester had the greatest biofilm eradicating effect and a superior activity than that of composition C composed of the individual components isosorbide and gallic acid.

### 7. Industrial applicability

The present invention provides compounds, formulations and methods for treating bacteria biofilms in both therapeutic and non-therapeutic settings.

## Claims

1. A compound according to Formula 1: 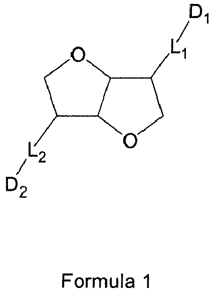 wherein
D₁ and D₂ are independently selected from -H, -OH, -COOR₁, -F, -Cl, Br, -O-C₁₋₆-alkyl,-CN, -CF₃, -SH, -NR₁R₂, a C1-C22 alkyl group, a C2-C22 alkenyl group, a C2-C22 alkynyl group, and a 5-10 membered aromatic hetero- or non-hetero mono- or bicyclic group,
wherein the C1-C22 alkyl group, the C2-C22 alkenyl group, and the C2-C22 alkynyl group is optionally substituted with 1 to 5 substituents independently selected from =O, -OH, - COOR₁, -F, -Cl, Br, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O, -OH, - COOR₁, -F, -Cl, Br, -C₁₋₆-alkyl, -C₂₋₆-alkenyl, -O-C₁₋₆-alkyl, -O-C₂₋₆-alkenyl, -CN, -CF₃, -SH and -NR₁R₂,
wherein the 5-10 membered aromatic group is optionally substituted with 1 to 5 substituents independently selected from =O, -OH, -COOR₁, -F, -Cl, -Br, -C₁₋₆-alkyl, -C₂₋₆-alkenyl, -O-C₁₋₆-alkyl, -O-C₂₋₆-alkenyl, -CN, -CF₃, -SH, -NR₁R₂, and phenyl, wherein said phenyl is optionally substituted with 1 to 3 substituents selected from =O, -OH, -COOR₁, -F, -Cl, -Br, -C₁₋₆-alkyl, -O-C₂₋₆-alkenyl, -O-C₁₋₆-alkyl, -O-C₂₋₆-alkenyl, -CN, -CF₃, -SH and -NR₁R₂,
wherein R₁ and R₂ in the above definitions are independently selected from H and C₁₋₆-alkyl;
and with the proviso that at least one of D₁ and D₂ is a 5-10 membered aromatic group substituted with one or more -OH groups and/or one or more -COOH groups;
and wherein L₁ and L₂ represent the bonds by which D₁ and D₂ are connected to the tetrahydrofuran rings, with L₁ and L₂ being independently selected from a direct single or direct double bond, an ester bond, an amide bond, an ether bond, a ketoester bond (i.e. α-ketoester, β-ketoester), a carbamate bond, a carbonate bond, a carbamide bond, a phosphate ester bond, a phosphate amine bond, a phosphonic ester bond, a phosphite ester bond, a phosphonate bond, a phosphinite bond, a phosphorane thiocarbonate bond, a dithiocarbonate bond, a thioether bond, a sulphite bond, a sulfonate bond, a sulfanate bond, a sulfonate bond, a silanol bond, a silyl ether bond, a hydroxylamine bond, an oxime bond, an imine bond, a boronate ester bond and an imino ether bond.

2. The compound according to claim 1, wherein D₁ and/or D₂ is a 5-10 membered aromatic group substituted with at least two -OH substituents, preferably with at least three -OH substituents.

3. The compound according to claims 1 or 2, wherein D₁ and/or D₂ is a 5-10 membered aromatic group substituted with at least one -COOH substituent.

4. The compound according to any one of claims 1 to 3, wherein D₁ and/or D₂ is a 5- or 6-membered aromatic group.

5. The compound according to any one of claims 1 to 4, wherein D₁ and/or D₂ is a benzene group.

6. The compound according to any one of claims 1 to 5, wherein D₁ and D₂ are the same.

7. The compound according to any one of claims 1 to 6, wherein L₁ and L₂ are independently an ester bond or an amide bond.

8. The compound according to any one of claims 1 to 7, having one of the following structures: 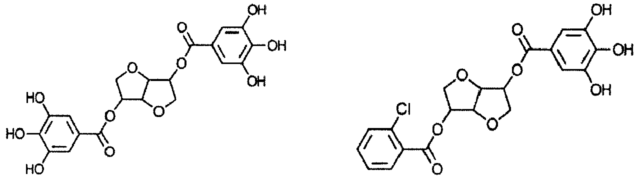 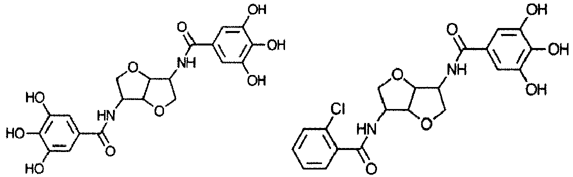 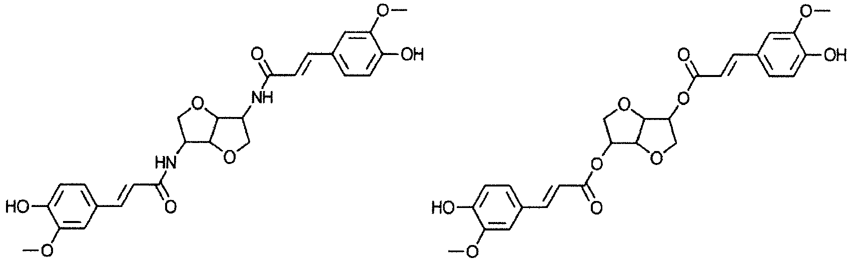 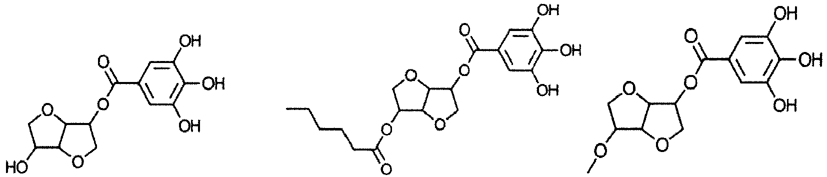 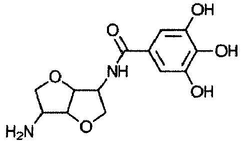 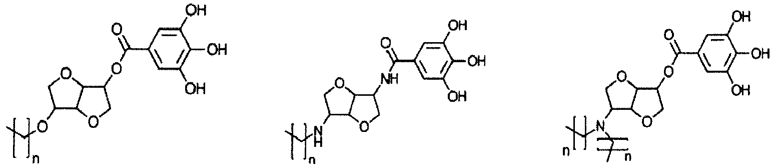 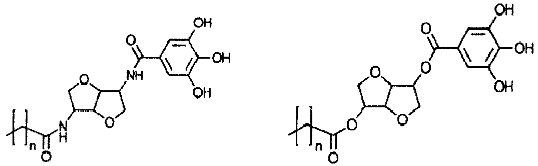 wherein 'n' in the above structures is an integer from 0 to 21.

9. A compound according to any one of claims 1 to 8, for use in a therapeutic method of preventing or hindering the formation of a bacterial biofilm in or on a human or animal.

10. Use of a compound according to any one of claims 1 to 8 in a non-therapeutic method of preventing or hindering the formation of a bacterial biofilm on an inanimate substrate.

11. The compound for use according to claim 9, or the use according to claim 10, wherein the bacterial biofilm comprises one or more of the following bacteria:
*Gram-positive: Actinomyces* spp., *Bacillus anthraeis, Bifidobacterium* spp., *Clostridium botulinum, Clostridium perfringens, Clostridium* spp., *Clostridium tetani, Corynebacterium diphtheriae, Corynebacterium jeikeium, Enterococcus faecalis, Enterococcus faecium, Erysipelothrix rhusiopathiae, Eubacterium* spp., *Gardnerella vaginalis, Gemella morbillorum, Leuconostoc* spp., *Mycobacterium abcessus, Mycobacterium avium complex, Mycobacterium chelonae, Mycobacterium fortuitum, Mycobacterium haemophilium, Mycobacterium kansasii, Mycobacterium leprae, Mycobacterium marinum, Mycobacterium scrofulaceum, Mycobacterium smegmatis, Mycobacterium terrae, Mycobacterium tuberculosis, Mycobacterium ulcerans, Nocardia* spp., *Peptococcus nigger, Peptostreptococcus* spp., *Proprionibacterium* spp., *Staphylococcus aureus, Staphylococcus auricularis, Staphylococcus capitis, Staphylococcus cohnii, Staphylococcus epidermidis, Staphylococcus haemolyticus, Staphylococcus hominis, Staphylococcus lugdanensis, Staphylococcus saccharolyticus, Staphylococcus saprophyticus, Staphylococcus schleiferi, Staphylococcus similans, Staphylococcus warneri, Staphylococcus xylosus, Streptococcus agalactiae* (group B *streptococcus*), *Streptococcus anginosus, Streptococcus bovis, Streptococcus canis, Streptococcus equi, Streptococcus milleri, Streptococcus mitior, Streptococcus mutans, Streptococcus pneunzoniae, Streptococcus pyogenes* (group A *streptococcus), Streptococcus salivarius,* and *Streptococcus sanguis;*
*Gram-negative: Acinetobacter calcoaceticus, Actinobacillus actinomycetemcomitans, Aeromonas hydrophila, Alcaligenes xylosoxidans, Bacteroides, Bacteroides fragilis, Bartonella bacilliformis, Bordetella* spp., *Borrelia burgdorferi, Branhamella catarrhalis, Brucella* spp., *Campylobacter* spp., *Chlamydia pneumoniae, Chlamydia psittaci, Chlamydia trachomatis, Chromobacterium violaceum, Citrobacter* spp., *Eikenella corrodens, Enterobacter aerogenes, Escherichia coli, Flavobacterium meningosepticum, Fusobacterium* spp., *Haemophilus influenzae, Haemophilus* spp., *Helicobacter pylori, Klebsiella* spp., *Legionella* spp., *Leptospira* spp., *Moraxella catarrhalis, Morganella morganii, Mycoplasma pneumoniae, Neisseria gonorrhoeae, Neisseria meningitidis, Pasteurella multocida, Plesiomonas shigelloides, Prevotella* spp., *Proteus* spp., *Providencia rettgeri, Pseudomonas aeruginosa, Pseudomonas* spp., *Rickettsia prowazekii, Rickettsia rickettsii, Rochalimaea* spp., *Salmonella* spp., *Salmonella typhi, Serratia marcescens, Shigella* spp., *Treponema carateum, Treponema pallidum, Treponema pallidum endemicum, Treponema pertenue, Veillonella* spp., *Vibrio cholerae, Vibrio vulnificus, Yersinia enterocolitica, Yersinia pestis*

12. A composition comprising:
a compound according to any one of claims 1 to 8; and
one or more antibiotics selected from iscapreomycins, such as capreomycin IA, capreomycin IB, capreomycin IIA and capreomycin IIB; carbomycins, such as carbomycin A; carumonam; cefaclor, cefadroxil, cefamandole, cefatrizine, cefazedone, cefazolin, cefbuperazone, cefcapene pivoxil, cefclidin, cefdinir, cefditoren, cefime, ceftamet, cefinenoxime, cefinetzole, cefminox, cefodizime, cefonicid, cefoperazone, ceforanide, cefotaxime, cefotetan, cefotiam, cefoxitin, cefpimizole, cefpiramide, cefpirome, cefprozil, cefroxadine, cefsulodin, ceftazidime, cefteram, ceftezole, ceftibuten, ceftiofur, ceftizoxime, ceftriaxone, cefuroxime, cefuzonam, cephalexin, cephalogycin, cephaloridine, cepholosporin C, cephalothin, cephapirin, cephamycins, such as cephamycin C, cephradine, chlortetracycline; chlarithromycin, clindamycin, clometocillin, clomocycline, cloxacillin, cyclacillin, danofloxacin, demeclocyclin, destomycin A, dicloxacillin, dicloxacillin, dirithromycin, doxycyclin, epicillin, erythromycin A, ethanbutol, fenbenicillin, flomoxef, florfenicol, floxacillin, flumequine, fortimicin A, fortimicin B, forfomycin, foraltadone, fusidic acid, gentamycin, glyconiazide, guamecycline, hetacillin, idarubicin, imipenem, isepamicin, josamycin, kanamycin, leumycins such as leumycin A1, lincomycin, lomefloxacin, loracarbef, lymecycline, meropenam, metampicillin, methacycline, methicillin, mezlocillin, micronaomicin, midecamycins such as midecamycin A1, mikamycin, minocycline, mitomycins such as mitomycin C, moxalactam, mupirocin, nafcillin, netilicin, norcardians such as norcardian A, oleandomycin, oxytetracycline, panipenam, pazufloxacin, penamecillin, penicillins such as penicillin G, penicillin N and penicillin 0, penillic acid, pentylpenicillin, peplomycin, phenethicillin, pipacyclin, piperacilin, pirlimycin, pivampicillin, pivcefalexin, porfiromycin, propiallin, quinacillin, ribostamycin, rifabutin, rifamide, rifampin, rifamycin SV, rifapentine, rifaximin, ritipenem, rekitamycin, rolitetracycline, rosaramicin, roxithromycin, sancycline, sisomicin, sparfloxacin, spectinomycin, streptozocin, sulbenicillin, sultamicillin, talampicillin, teicoplanin, temocillin, tetracycline, thostrepton, tiamulin, ticarcillin, tigemonam, tilmicosin, tobramycin, tropospectromycin, trovafloxacin, tylosin, and vancomycin, as well as pharmaceutically acceptable salts, esters, prodrugs and protected forms thereof.

13. The composition according to claim 11, for use in a method of preventing or treating a bacterial infection in a patient.

14. A medical device comprising a compound according to any one of claims 1 to 8.

15. The medical device according to claim 15 wherein the medical device is selected from the group consisting of grafts, membranes, tubes, connectors, surgical instruments, intra-aortic balloons, stents (including drug-eluting vascular stents), blood bags, catheters, sutures, prostheses, heart valves, tissue adhesives, cardiac pacemaker leads, artificial organs, lenses for the eye, blood handling equipment, apheresis equipment, (bio)sensors, dental devices, skin patches, wound dressings (including drug-eluting wound dressings), implantable devices, tampons, bandages, drug delivery systems, bodily implants, and protein-eluting scaffolds for tissue regeneration.
